# EUROPÄISCHE PATENTSCHRIFT

(11) **EP 4 197 633 B1**
(45) Veröffentlichungstag und Bekanntmachung des Hinweises auf die Patenterteilung: **07.08.2024**
(21) Anmeldenummer: 21215383.7
(22) Anmeldetag: 17.12.2021
(51) Int. Cl.: B01J 31/02, B01J 31/24

(54) **PT-KOMPLEX-GEMISCH**
PT COMPLEX MIXTURE
MÉLANGE DE COMPLEXE PT

(43) Veröffentlichungstag der Anmeldung: 21.06.2023
(73) Patentinhaber: Evonik Oxeno GmbH & Co. KG, 45772 Marl (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- CN-A- 113 461 737
- BOTTEGHI C. ET AL: "Synthesis of 2-chromanol by hydroformylation of 2-hydroxystyrene derivatives", vol. 200, no. 1-2, 1 June 2003 (2003-06-01), NL, pages 147 - 156, XP055924922, ISSN: 1381-1169, Retrieved from the Internet <URL:https://www.sciencedirect.com/science/article/pii/S1381116903001213/pdfft?md5=2768ecee6bc7030cbc06418d714433c4&pid=1-s2.0-S1381116903001213-main.pdf> DOI: 10.1016/S1381-1169(03)00121-3
- BEHR A ET AL: "Selective catalytic formation of unsaturated amino acids from petrochemicals and carbon dioxide-Application of high-throughput catalyst screening", JOURNAL OF MOLECULAR CATALYSIS A: CHEMICAL, ELSEVIER, AMSTERDAM, NL, vol. 287, no. 1-2, 15 May 2008 (2008-05-15), pages 95 - 101, XP022667252, ISSN: 1381-1169, [retrieved on 20080219], DOI: 10.1016/J.MOLCATA.2008.02.011

## Beschreibung

Die vorliegende Erfindung betrifft eine Pt-Komplex-Gemisch, sowie dessen Einsatz in der Katalyse.

In C. Botteghi et al., Journal of Molecular Catalysis A: Chemical 200, (2003), 147-156 wird der Einsatz von Pt(Xantphos)Cl₂ zur Hydroformylierung von 2-Tosyloxystyrol beschrieben.

Der vorliegende Erfindung lag die Aufgabe zugrunde, einen neues Katalysatorsystem bereitzustellen. Mit diesem Katalysatorsystem sollen Olefine zu Alkoholen umgesetzt werden. Hierbei soll eine gute Ausbeute erzielt werden.

Diese Aufgabe wird gelöst durch eine Komplex-Gemisch gemäß Anspruch 1.

Komplex-Gemisch umfassen:
a) einen ersten Komplex (**K1**) umfassend:
   a1) Pt;
   a2) einen Liganden entsprechend der Formel (I): 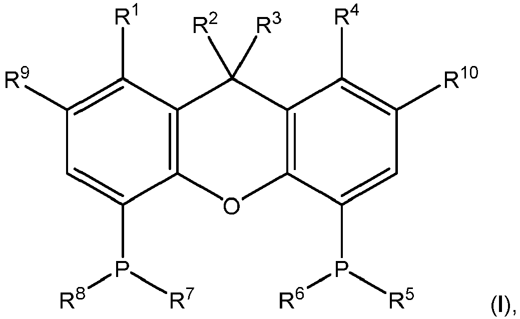 wobei R¹, R², R³, R⁴, R⁵, R⁶, R⁷, R⁸, R⁹, R¹⁰ ausgewählt sind aus: -H, -(C₁-C₁₂)-Alkyl, -(C₆-C₂₀)-Aryl, und für den Fall, dass R¹, R², R³, R⁴, R⁵, R⁶, R⁷, R⁸, R⁹, R¹⁰ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
   a3) mindesten einen lod-Liganden oder Brom-Liganden;
b) einen zweiten Komplex (**K2**) umfassend:
   b1) Pt;
   b2) einen Liganden entsprechend der Formel (**II**): 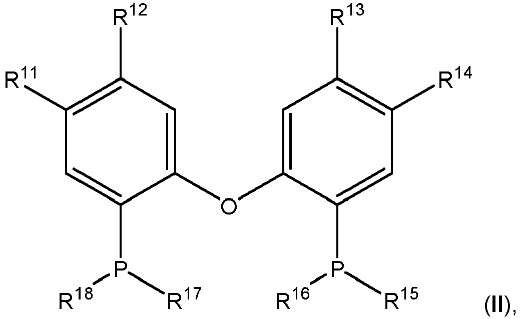 wobei R¹¹, R¹², R¹³, R¹⁴, R¹⁵, R¹⁶, R¹⁷, R¹⁸ ausgewählt sind aus: -H, -(C₁-C₁₂)-Alkyl, -(C₆-C₂₀)-Aryl; und für den Fall, dass R¹¹, R¹², R¹³, R¹⁴, R¹⁵, R¹⁶, R¹⁷, R¹⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
   b3) mindesten einen lod-Liganden oder Brom-Liganden;
      wobei das Komplex-Gemisch ein Verhältnis von (**K1**) zu (**K2**) von 1:1 aufweist.

Das Merkmal a3) beziehungsweise b3) ist so zu lesen, dass mindesten ein lod-Liganden oder Brom-Liganden vorhanden sein muss. Es ist auch möglich, dass ein Teil des Komplexes **K1** lod-Liganden und ein anderer Teil Brom-Liganden aufweist. Dies kann vor allem dann der Fall sein, wenn die Komplexe in situ, also erst im Reaktionsgemisch, ausgebildet werden und dem Reaktionsgemisch ein lodid und ein Bromid zugegeben wird. Koordiniert mehr als ein Halogen an das Metall (Pt), so können diese auch unterschiedlich sein.

Entsprechendes gilt auch für **K2.**

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Ausführungsform sind R², R³, R⁵, R⁶, R⁷, R⁸ ausgewählt aus: -(C₁-C₁₂)-Alkyl, -(C₆-C₂₀)-Aryl.

In einer Ausführungsform stehen R⁵, R⁶, R⁷, R⁸ für -(C₆-C₂₀)-Aryl.

In einer Ausführungsform stehen R⁵, R⁶, R⁷, R⁸ für -Ph

In einer Ausführungsform stehen R² und R³ für -(C₁-C₁₂)-Alkyl.

In einer Ausführungsform stehen R² und R³ für -CH₃.

In einer Ausführungsform stehen R¹ und R⁴ für -H.

In einer Ausführungsform stehen R⁹ und R¹⁰ für -H.

In einer Ausführungsform weist der Liganden (**I**) die Struktur (**1**) auf:

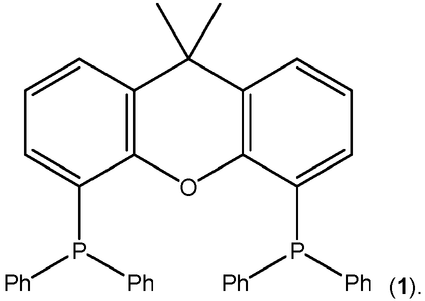

In einer Ausführungsform stehen R¹⁵, R¹⁶, R¹⁷, R¹⁸ für -(C₆-C₂₀)-Aryl.

In einer Ausführungsform stehen R¹⁵, R¹⁶, R¹⁷, R¹⁸ für -Ph.

In einer Ausführungsform stehen R¹¹ und R¹⁴ für-H.

In einer Ausführungsform stehen R¹² und R¹³ für -H.

In einer Ausführungsform weist der Liganden (**II**) die Struktur (**2**) auf:

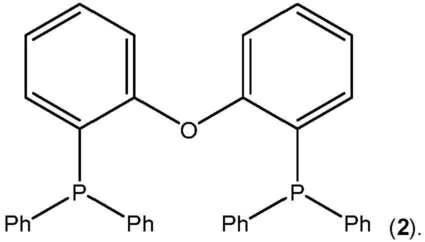

In einer Ausführungsform weist der Komplex (**K1**) genau einen Liganden entsprechend der Formel (I) auf.

In einer Ausführungsform weist der Komplex (**K2**) genau einen Liganden entsprechend der Formel (**II**) auf.

In einer Ausführungsform weist das Komplex-Gemisch ein Verhältnis von (**K1**) zu (**K2**) von 1:1 auf.

In einer Ausführungsform weist das Komplex-Gemisch lod und Brom auf.

Neben dem Komplex-Gemisch an sich, wird auch ein Verfahren beansprucht, in welchem das Komplex-Gemisch als Katalysatorsystem zur Katalyse eingesetzt wird.

Verfahren umfassend die Verfahrensschritte:
A) Vorlegen eines Olefins;
B) Zugabe eines Komplex-Gemisches wie zuvor beschrieben, oder dessen Komponenten:
   B1) eine Pt-Verbindung;
   B2) einen Liganden entsprechend der Formel (**I**): 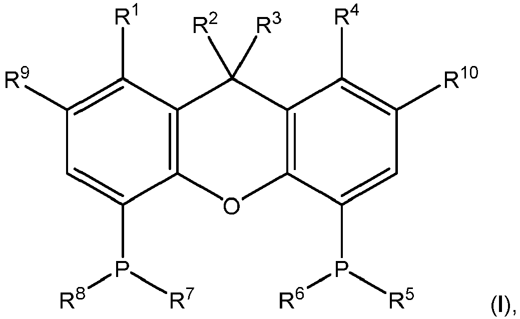 wobei R¹, R², R³, R⁴, R⁵, R⁶, R⁷, R⁸, R⁹, R¹⁰ ausgewählt sind aus: -H, -(C₁-C₁₂)-Alkyl, -(C₆-C₂₀)-Aryl, und für den Fall, dass R¹, R², R³, R⁴, R⁵, R⁶, R⁷, R⁸, R⁹, R¹⁰ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
   B3) einen Liganden entsprechend der Formel (**II**): 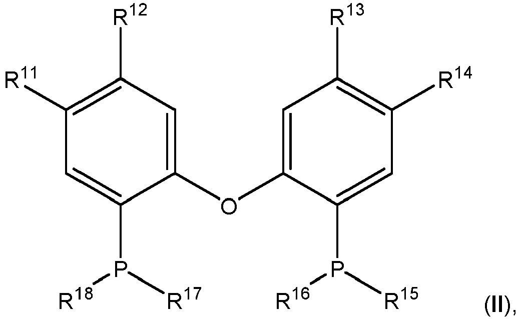 wobei R¹¹, R¹², R¹³, R¹⁴, R¹⁵, R¹⁶, R¹⁷, R¹⁸ ausgewählt sind aus: -H, -(C₁-C₁₂)-Alkyl, -(C₆-C₂₀)-Aryl; und für den Fall, dass R¹¹, R¹², R¹³, R¹⁴, R¹⁵, R¹⁶, R¹⁷, R¹⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
   B4) mindesten eine lod-Verbindung oder eine Brom-Verbindung; wobei das Komplex-Gemisch ein Verhältnis von (**K1**) zu (**K2**) von 1:1 aufweist;
C) Zuführen von CO und H₂;
D) Erwärmen des Reaktionsgemisches aus A) bis C), wobei das Olefin zu einem Alkohol umgesetzt wird.

Alle Ausführungen bezüglich Ausführungsformen des Komplex-Gemisches gelten analog für das Komplex-Gemisch bzw. deren Komponenten im Verfahren.

In einer Variante des Verfahrens umfasst dieses den zusätzlichen Verfahrensschritt C'): C') Zugabe eines Lösungsmittels.

In einer Variante des Verfahrens ist das Lösungsmittel ausgewählt aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

In einer Variante des Verfahrens ist das Lösungsmittel ausgewählt aus: THF, DCM, ACN, Heptan, DMF, Toluol, Texanol.

In einer Variante des Verfahrens ist die Pt-Verbindung in Verfahrensschritt B1) ausgewählt ist aus: Pt(II)I₂, Pt(II)Br₂, Pt(IV)I₄, Pt(IV)Br₄, Diphenyl(1,5-COD)Pt(II), Pt(II)(acac)₂, Pt(0)(PPh₃)₄, Pt(0)(DVTS)-Lösung (CAS:68478-92-2), Pt(0)(Ethylen)(PPh₃)₂, Pt(II)Br₂(COD), Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂.

In einer Variante des Verfahrens ist die Pt-Verbindung in Verfahrensschritt B1) ausgewählt ist aus: Pt(II)I₂, Pt(II)Br₂.

In einer Variante des Verfahrens ist die lod-Verbindung beziehungsweise die Brom-Verbindung in Verfahrensschritt B4) ausgewählt aus: Alkalihalogenid, Erdalkalihalogenid, NH₄X, Alkylammoniumhalogenid, Dialkylhalogenid, Trialkylhalogenid, Tetraalkylhalogenid, Cycloalkylammoniumhalogenid.

In einer Variante des Verfahrens ist die lod-Verbindung oder Brom-Verbindung in Verfahrensschritt B4) ausgewählt sind aus: Pt(II)I₂, Pt(II)Br₂.

In einer Variante des Verfahrens erfolgt das Zuführen von CO und H₂ bei einem Druck in einem Bereich von 1 MPa (10 bar) bis 6 MPa (60 bar).

In einer Variante des Verfahrens erfolgt das Zuführen von CO und H₂ bei einem Druck in einem Bereich von 1 MPa (20 bar) bis 6 MPa (50 bar).

In einer Variante des Verfahrens erfolgt das Erwärmen auf eine Temperatur im Bereich von 50 °C bis 200°C.

In einer Variante des Verfahrens erfolgt das Erwärmen auf eine Temperatur im Bereich von 80 °C bis 170°C.

In einer Variante des Verfahrens ist das Olefin ausgewählt aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

Im Folgenden soll die Erfindung anhand von Ausführungsbeispielen näher erläutert werden.

### Versuchsbeschreibung

Ein Vial wurde mit PtBr₂ und PtI₂, Ligand (**1**) und Ligand (**2**), und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und 1-Octen in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 20 h bei 150 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Variation des Halogens

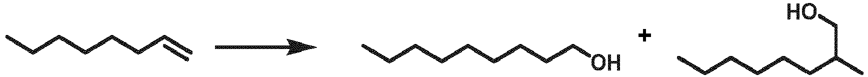

Reaktionsbedingungen:
5.0 mmol 1-Octen, 2.0 mol% PtX₂ (X = Halogen), 1.1 Äquivalente DPEPhos (2), 1.1 Äquivalente Xantphos (1), Lösungsmittel: Toluol, p(CO/H₂): 40 bar, T: 150 °C, t: 20 h.

### Ausbeute an Alkohol:

PtBr₂: 82%
Ptl₂: 48%
PtCl₂: 3 %

Wie die Versuchsergebnisse zeigen, wird die Aufgabe durch das erfindungsgemäße Komplex-Gemisch gelöst.

## Patentansprüche

1. Komplex-Gemisch umfassen:
a) einen ersten Komplex (**K1**) umfassend:
a1) Pt;
a2) einen Liganden entsprechend der Formel (I): 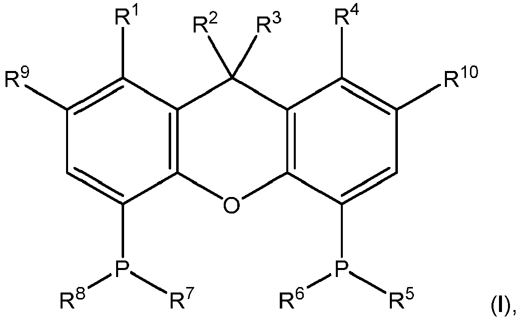 wobei R¹, R², R³, R⁴, R⁵, R⁶, R⁷, R⁸, R⁹, R¹⁰ ausgewählt sind aus: -H, -(C₁-C₁₂)-Alkyl, -(C₆-C₂₀)-Aryl, und für den Fall, dass R¹, R², R³, R⁴, R⁵, R⁶, R⁷, R⁸, R⁹, R¹⁰ für-(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
a3) mindesten einen lod-Liganden oder Brom-Liganden;
b) einen zweiten Komplex (**K2**) umfassend:
b1) Pt;
b2) einen Liganden entsprechend der Formel (**II**): 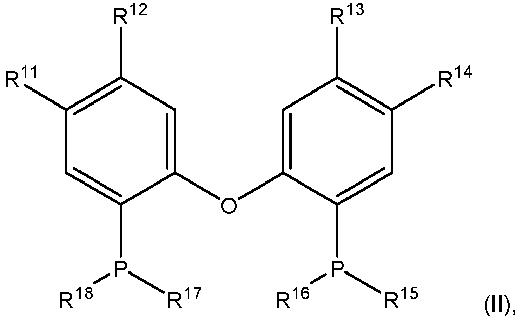
wobei R¹¹, R¹², R¹³, R¹⁴, R¹⁵, R¹⁶, R¹⁷, R¹⁸ ausgewählt sind aus: -H, -(C₁-C₁₂)-Alkyl, -(C₆-C₂₀)-Aryl; und für den Fall, dass R¹¹, R¹², R¹³, R¹⁴, R¹⁵, R¹⁶, R¹⁷, R¹⁸ für-(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
b3) mindesten einen lod-Liganden oder Brom-Liganden;
wobei das Komplex-Gemisch ein Verhältnis von (**K1**) zu (**K2**) von 1:1 aufweist.

2. Komplex-Gemisch nach Anspruch 1,
wobei R², R³, R⁵, R⁶, R⁷, R⁸ ausgewählt sind aus: -(C₁-C₁₂)-Alkyl, -(C₆-C₂₀)-Aryl.

3. Komplex-Gemisch nach einem der Ansprüche 1 oder 2,
wobei R² und R³ für -(C₁-C₁₂)-Alkyl stehen.

4. Komplex-Gemisch nach einem der Ansprüche 1 bis 3,
wobei der Liganden (**I**) die Struktur (**1**) aufweist:

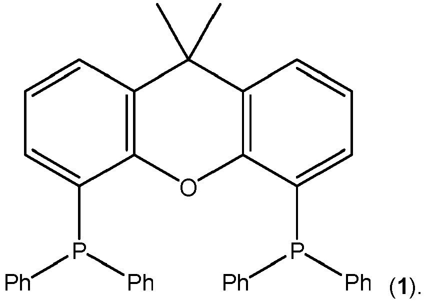

5. Komplex-Gemisch nach einem der Ansprüche 1 bis 4, wobei R¹⁵, R¹⁶, R¹⁷, R¹⁸ für -(C₆-C₂₀)-Aryl stehen.

6. Komplex-Gemisch nach einem der Ansprüche 1 bis 5, wobei R¹¹ und R¹⁴für-H stehen.

7. Komplex-Gemisch nach einem der Ansprüche 1 bis 6, wobei R¹² und R¹³ für -H stehen.

8. Komplex-Gemisch nach einem der Ansprüche 1 bis 7, wobei der Liganden (**II**) die Struktur (**2**) aufweist:

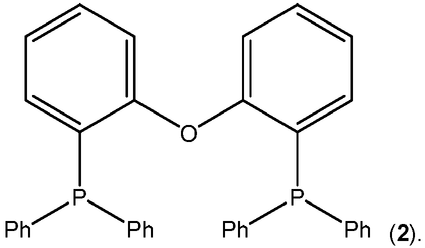

9. Komplex-Gemisch nach einem der Ansprüche 1 bis 8,
wobei das Komplex-Gemisch lod und Brom aufweist.

10. Verfahren umfassend die Verfahrensschritte:
A) Vorlegen eines Olefins;
B) Zugabe eines Komplex-Gemisches nach einem der Ansprüche 1 bis 9, oder dessen Komponenten:
B1) eine Pt-Verbindung;
B2) einen Liganden entsprechend der Formel (**I**): 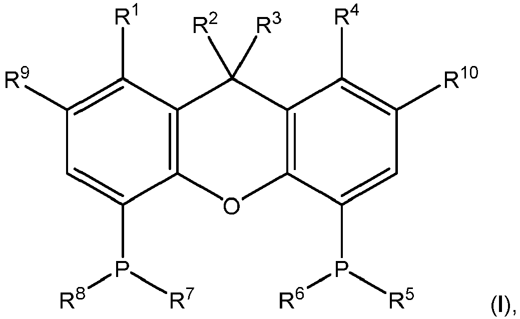 wobei R¹, R², R³, R⁴, R⁵, R⁶, R⁷, R⁸, R⁹, R¹⁰ ausgewählt sind aus: -H, -(C₁-C₁₂)-Alkyl, -(C₆-C₂₀)-Aryl, und für den Fall, dass R¹, R², R³, R⁴, R⁵, R⁶, R⁷, R⁸, R⁹, R¹⁰ für-(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
B3) einen Liganden entsprechend der Formel (**II**): 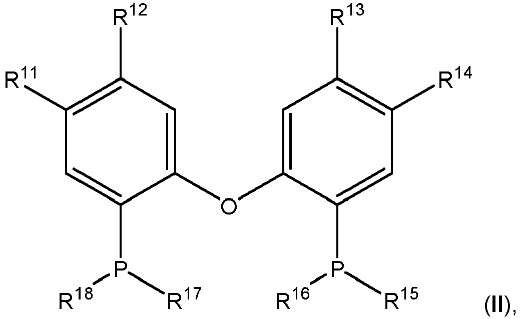 wobei R¹¹, R¹², R¹³, R¹⁴, R¹⁵, R¹⁶, R¹⁷, R¹⁸ ausgewählt sind aus: -H, -(C₁-C₁₂)-Alkyl, -(C₆-C₂₀)-Aryl; und für den Fall, dass R¹¹, R¹², R¹³, R¹⁴, R¹⁵, R¹⁶, R¹⁷, R¹⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
B4) mindesten eine lod-Verbindung oder eine Brom-Verbindung;
wobei das Komplex-Gemisch ein Verhältnis von (**K1**) zu (**K2**) von 1:1 aufweist;
C) Zuführen von CO und H₂;
D) Erwärmen des Reaktionsgemisches aus A) bis C), wobei das Olefin zu einem Alkohol umgesetzt wird.

11. Verfahren nach Anspruch 10,
wobei das Verfahren den zusätzlichen Verfahrensschritt C') umfasst: C') Zugabe eines Lösungsmittels.

12. Verfahren nach einem der Ansprüche 10 oder 11, wobei die Pt-Verbindung in Verfahrensschritt B1) ausgewählt ist aus: Pt(II)I₂, Pt(II)Br₂, Pt(IV)I₄, Pt(IV)Br₄, Diphenyl(1,5-COD)Pt(II), Pt(II)(acac)₂, Pt(0)(PPh₃)₄, Pt(0)(DVTS)-Lösung (CAS:68478-92-2), Pt(0)(Ethylen)(PPh₃)₂, Pt(II)Br₂(COD), Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂.

13. Verfahren nach einem der Ansprüche 10 bis 12,
wobei die lod-Verbindung oder Brom-Verbindung in Verfahrensschritt B4) ausgewählt sind aus: Pt(II)I₂, Pt(II)Br₂.

14. Verfahren nach einem der Ansprüche 10 bis 13,
wobei das Zuführen von CO und H₂ bei einem Druck in einem Bereich von 1 MPa (10 bar) bis 6 MPa (60 bar) erfolgt.

## Claims

1. Complex mixture comprising:
a) a first complex (K1) comprising:
a1) Pt;
a2) a ligand corresponding to formula (I): 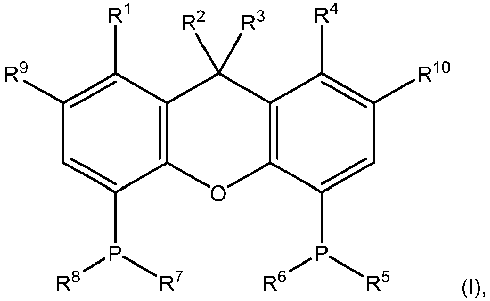 where R¹, R², R³, R⁴, R⁵, R⁶, R⁷, R⁸, R⁹, R¹⁰ are selected from: -H, -(C₁-C₁₂)-alkyl, -(C₆-C₂₀)-aryl, and, if R¹, R², R³, R⁴, R⁵, R⁶, R⁷, R⁸, R⁹, R¹⁰ are -(C₆-C₂₀)-aryl, the aryl ring may have substituents selected from: -(C₁-C₁₂)-alkyl, -O-(C₁-C₁₂)-alkyl;
a3) at least one iodine ligand or bromine ligand;
b) a second complex (K2) comprising:
b1) Pt;
b2) a ligand corresponding to formula (II): 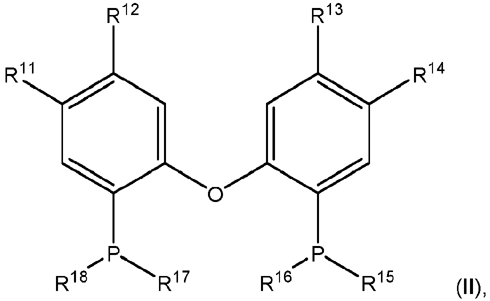 where R¹¹, R¹², R¹³, R¹⁴, R¹⁵, R¹⁶, R¹⁷, R¹⁸ are selected from: -H, -(C₁-C₁₂)-alkyl, -(C₆-C₂₀)-aryl; and, if R¹¹, R¹², R¹³, R¹⁴, R¹⁵, R¹⁶, R¹⁷, R¹⁸ are - (C₆-C₂₀)-aryl, the aryl ring may have substituents selected from: -(C₁-C₁₂)-alkyl, -O-(C₁-C₁₂)-alkyl;
b3) at least one iodine ligand or bromine ligand; wherein the complex mixture has a ratio of (K1) to (K2) of 1:1.

2. Complex mixture according to Claim 1,
wherein R², R³, R⁵, R⁶, R⁷, R⁸ are selected from: -(C₁-C₁₂)-alkyl, -(C₆-C₂₀)-aryl.

3. Complex mixture according to either of Claims 1 and 2,
wherein R² and R³ are -(C₁-C₁₂)-alkyl.

4. Complex mixture according to any of Claims 1 to 3, wherein the ligand (I) has the structure (1):

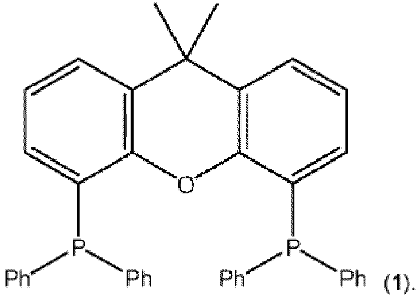

5. Complex mixture according to any of Claims 1 to 4, wherein R¹⁵, R¹⁶, R¹⁷, R¹⁸ are - (C₆-C₂₀)-aryl.

6. Complex mixture according to any of Claims 1 to 5, wherein R¹¹ and R¹⁴ are -H.

7. Complex mixture according to any of Claims 1 to 6, wherein R¹² and R¹³ are -H.

8. Complex mixture according to any of Claims 1 to 7, wherein the ligand (II) has the structure (2):

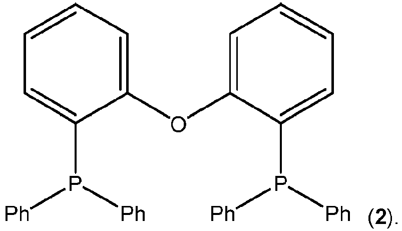

9. Complex mixture according to any of Claims 1 to 8, wherein the complex mixture comprises iodine and bromine.

10. Process comprising the process steps of:
A) initially charging an olefin;
B) adding a complex mixture according to any of Claims 1 to 9, or the components thereof:
B1) a Pt compound;
B2) a ligand corresponding to formula (I): 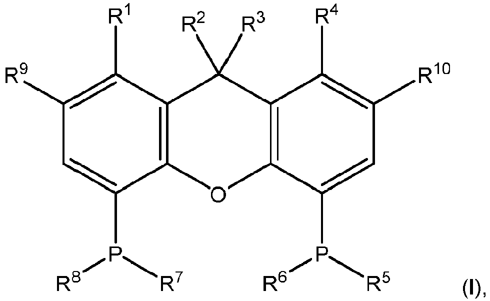 where R¹, R², R³, R⁴, R⁵, R⁶, R⁷, R⁸, R⁹, R¹⁰ are selected from: -H, -(C₁-C₁₂)-alkyl, -(C₆-C₂₀)-aryl, and, if R¹, R², R³, R⁴, R⁵, R⁶, R⁷, R⁸, R⁹, R¹⁰ are -(C₆-C₂₀)-aryl, the aryl ring may have substituents selected from: -(C₁-C₁₂)-alkyl, -O-(C₁-C₁₂)-alkyl;
B3) a ligand corresponding to formula (II): 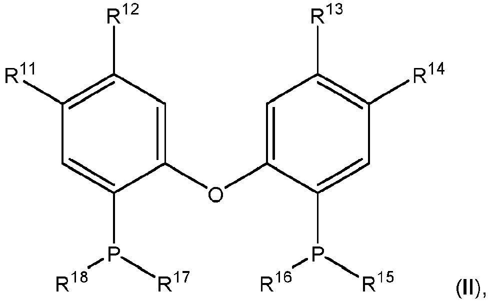 where R¹¹, R¹², R¹³, R¹⁴, R¹⁵, R¹⁶, R¹⁷, R¹⁸ are selected from: -H, -(C₁-C₁₂)-alkyl, -(C₆-C₂₀)-aryl; and, if R¹¹, R¹², R¹³, R¹⁴, R¹⁵, R¹⁶, R¹⁷, R¹⁸ are -(C₆-C₂₀)-aryl, the aryl ring may have substituents selected from: -(C₁-C₁₂)-alkyl, -O-(C₁-C₁₂)-alkyl;
B4) at least one iodine compound or one bromine compound;
wherein the complex mixture has a ratio of (K1) to (K2) of 1:1;
C) feeding in CO and H₂;
D) heating the reaction mixture from A) to C), with conversion of the olefin to an alcohol.

11. Process according to Claim 10,
wherein the process comprises the additional process step C'):
C') adding a solvent.

12. Process according to either of Claims 10 and 11, wherein the Pt compound in process step B1) is selected from: Pt(II)I₂, Pt(II)Br₂, Pt(IV)I₄, Pt(IV)Br₄, diphenyl (1,5-COD)Pt(II), Pt(II)(acac)₂, Pt(0)(PPh₃)₄, Pt(0) (DVTS) solution (CAS:68478-92-2), Pt(0)(ethylene)(PPh₃)₂, Pt(II)Br₂(COD), tris(benzylideneacetone)Pt(0), Pt(II) (OAC)₂ solution, Pt(0)(t-Bu)₂, Pt(II)(COD)Me₂, Pt(II)(COD)I₂, Pt(IV)IMe₃, Pt(II)(hexafluoroacetylacetonate)₂.

13. Process according to any of Claims 10 to 12, wherein the iodine compound or bromine compound in process step B4) are selected from: Pt(II)I₂, Pt(II)Br₂.

14. Process according to any of Claims 10 to 13, wherein CO and H₂ are fed in at a pressure in a range from 1 MPa (10 bar) to 6 MPa (60 bar).

## Revendications

1. Mélange de complexes, comprenant :
a) un premier complexe (K1) comprenant :
a1) du Pt ;
a2) un ligand selon la formule (I) : 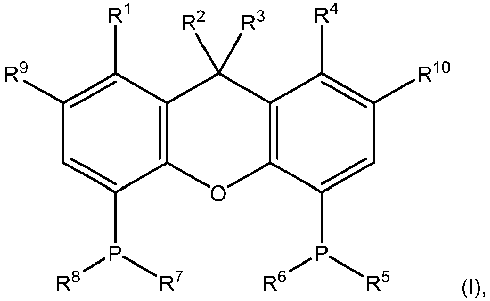 dans laquelle R¹, R², R³, R⁴, R⁵, R⁶, R⁷, R⁸, R⁹, R¹⁰ sont choisis parmi : -H, -(C₁-C₁₂)-alkyle, -(C₆-C₂₀)-aryle ; et pour le cas où R¹, R², R³, R⁴, R⁵, R⁶, R⁷, R⁸, R⁹, R¹⁰ représentent -(C₆-C₂₀)-aryle, le cycle aryle peut présenter des substituants qui sont choisis parmi : -(C₁-C₁₂) -alkyle, -O-(C₁-C₁₂)-alkyle ;
a3) au moins un ligand iode ou ligand brome ;
b) un deuxième complexe (K2) comprenant :
b1) du Pt ;
b2) un ligand selon la formule (II) : 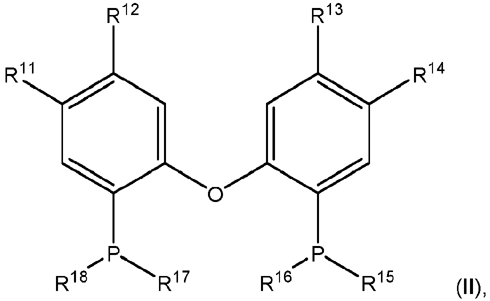 dans laquelle R¹¹, R¹², R¹³, R¹⁴, R¹⁵, R¹⁶, R¹⁷, R¹⁸ sont choisis parmi : -H, -(C₁-C₁₂)-alkyle, -(C₆-C₂₀)-aryle ; et pour le cas où R¹¹, R¹², R¹³, R¹⁴, R¹⁵, R¹⁶, R¹⁷, R¹⁸ représentent -(C₆-C₂₀)-aryle, le cycle aryle peut présenter des substituants qui sont choisis parmi : -(C₁-C₁₂) -alkyle, -O-(C₁-C₁₂)-alkyle ;
b3) au moins un ligand d'iode ou ligand de brome ; le mélange de complexes présentant un rapport de (K1) à (K2) de 1:1.

2. Mélange de complexes selon la revendication 1,
R², R³, R⁵, R⁶, R⁷, R⁸ étant choisis parmi : -(C₁-C₁₂)-alkyle, -(C₆-C₂₀)-aryle.

3. Mélange de complexes selon l'une des revendications 1 ou 2,
R² et R³ représentant -(C₁-C₁₂)-alkyle.

4. Mélange de complexes selon l'une des revendications 1 à 3,
le ligand (I) présentant la structure (1) :

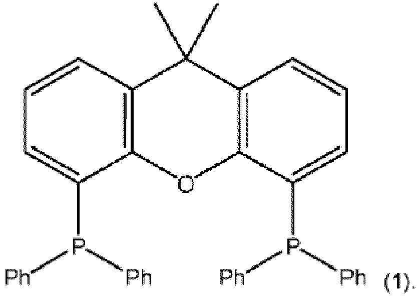

5. Mélange de complexes selon l'une des revendications 1 à 4,
R¹⁵, R¹⁶, R¹⁷, R¹⁸ représentant -(C₆-C₂₀)-aryle.

6. Mélange de complexes selon l'une des revendications 1 à 5,
R¹¹ et R¹⁴ représentant -H.

7. Mélange de complexes selon l'une des revendications 1 à 6,
R¹² et R¹³ représentant -H.

8. Mélange de complexes selon l'une des revendications 1 à 7,
le ligand (II) présentant la structure (2) :

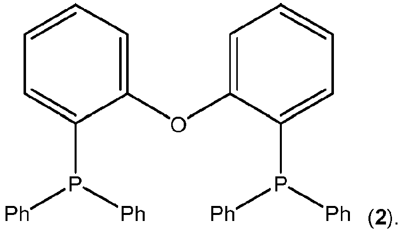

9. Mélange de complexes selon l'une des revendications 1 à 8,
le mélange de complexes présentant de l'iode et du brome.

10. Procédé comprenant les étapes de procédé :
A) disposition préalable d'une oléfine ;
B) ajout d'un mélange de complexes selon l'une des revendications 1 à 9 ou de ses composants :
B1) un composé de Pt ;
B2) un ligand selon la formule (I) : 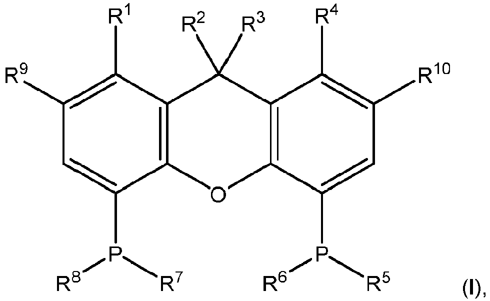 dans laquelle R¹, R², R³, R⁴, R⁵, R⁶, R⁷, R⁸, R⁹, R¹⁰ sont choisis parmi : -H, -(C₁-C₁₂)-alkyle, -(C₆-C₂₀)-aryle ; et pour le cas où R¹, R², R³, R⁴, R⁵, R⁶, R⁷, R⁸, R⁹, R¹⁰ représentent -(C₆-C₂₀)-aryle, le cycle aryle peut présenter des substituants qui sont choisis parmi : -(C₁-C₁₂) -alkyle, -O-(C₁-C₁₂)-alkyle ;
B3) un ligand selon la formule (II) : 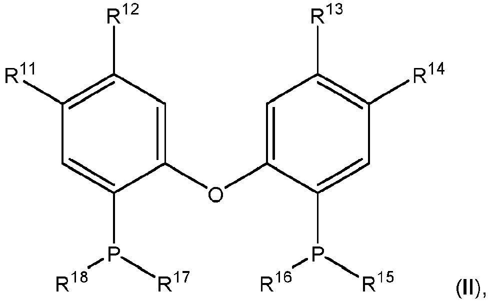 dans laquelle R¹¹, R¹², R¹³, R¹⁴, R¹⁵, R¹⁶, R¹⁷, R¹⁸ sont choisis parmi : -H, -(C₁-C₁₂)-alkyle, -(C₆-C₂₀)-aryle ; et pour le cas où R¹¹, R¹², R¹³, R¹⁴, R¹⁵, R¹⁶, R¹⁷, R¹⁸ représentent -(C₆-C₂₀)-aryle, le cycle aryle peut présenter des substituants qui sont choisis parmi : -(C₁-C₁₂) -alkyle, -O-(C₁-C₁₂)-alkyle ;
B4) au moins un ligand iode ou ligand brome ;
le mélange de complexes présentant un rapport de (K1) à (K2) de 1:1 ;
C) introduction de CO et de H₂ ;
D) chauffage du mélange réactionnel provenant de A) à C), l'oléfine étant transformée en alcool.

11. Procédé selon la revendication 10,
le procédé comprenant l'étape de procédé C') supplémentaire :
C') ajout d'un solvant.

12. Procédé selon l'une des revendications 10 ou 11, le composé de Pt dans l'étape de procédé B1) étant choisi parmi : Pt(II)I₂, Pt(II)Br₂, Pt(IV)I₄, Pt(IV)Br₄, diphényl(1,5-COD)Pt(II), Pt(II)(acac)₂, Pt(0)(PPh₃)₄, solution de Pt(0) (DVTS) (CAS:68478-92-2), Pt(0)(éthylène)(PPh₃)₂, Pt(II)Br₂(COD), tris(benzylidénacétone)Pt(0), solution de Pt(II)(OAc)₂, Pt(0)(t-Bu)₂, Pt(II)(COD)Me₂, Pt(II)(COD)I₂, Pt(IV)IMe₃, Pt(II)(hexafluoroacétylacétonate)₂.

13. Procédé selon l'une des revendications 10 à 12, le composé d'iode ou le composé de brome dans l'étape de procédé B4) étant choisis parmi : Pt(II)I₂, Pt(II)Br₂.

14. Procédé selon l'une des revendications 10 à 13, l'introduction de CO et de H₂ ayant lieu à une pression dans la plage de 1 MPa (10 bars) à 6 MPa (60 bars).
